Europäisches Patentamt

European Patent Office

Office européen des brevets

(19)

(11) Veröffentlichungsnummer: **0 012 968**
A1

(12) # EUROPÄISCHE PATENTANMELDUNG

(21) Anmeldenummer: 79105213.7

(22) Anmeldetag: 17.12.79

(51) Int. Cl.³: **C 07 C 69/15**
C 07 C 67/055

(30) Priorität: 21.12.78 DE 2855283

(43) Veröffentlichungstag der Anmeldung:
09.07.80 Patentblatt 80/14

(84) Benannte Vertragsstaaten:
BE DE FR GB IT NL

(71) Anmelder: HOECHST AKTIENGESELLSCHAFT
Zentrale Patentabteilung Postfach 80 03 20
D-6230 Frankfurt/Main 80(DE)

(72) Erfinder: Fernholz, Hans, Prof. Dr.
Taunusstrasse 57
D-6233 Kelkheim (Taunus)(DE)

(72) Erfinder: Schmidt, Hans-Joachim, Dr.
Am Burgenblick 6
D-6240 Königstein/Taunus(DE)

(72) Erfinder: Roscher, Günter, Dr.
Altkönigstrasse 7
D-6233 Kelkheim (Taunus)(DE)

(54) Verfahren zur Herstellung von Vinylacetat.

(57) Vinylacetat wird in der Gasphase aus Ethylen, Essigsäure und Sauerstoff an palladiumhaltigen Trägerkatalysatoren hergestellt. Dabei ist das molare Verhältnis von Essigsäure zu Sauerstoff im eingesetzten Gasgemisch größer als 2 : 1.

EP 0 012 968 A1

Croydon Printing Company Ltd

HOECHST AKTIENGESELLSCHAFT   HOE 78/F 282          /Dr.MA/ss

Verfahren zur Herstellung von Vinylacetat

Bei der bekannten Herstellung von Vinylacetat in der Gasphase durch Umsetzung von Ethylen, Essigsäure und mole-kularem Sauerstoff an palladiumhaltigen Trägerkatalysa-toren werden i. allg. Gasgemische eingesetzt, die weniger als 8 Vol.-% Sauerstoff (bezogen auf das essigsäurefreie Gasgemisch) enthalten und in denen die Konzentrationsverhältnisse so gewählt sind, daß das Reaktionsgemisch außerhalb der bekannten Explosionsgrenzen liegt. Nach den Literaturangaben können Gasgemische verwendet werden, in denen auf 1 Mol Essigsäure 0,25 bis 2 Mol Sauerstoff und 1 bis 100 Mol Ethylen entfallen. Nach der japanischen Patentanmeldung 3969/65 ist es vorteilhaft, wenn das Molverhältnis Essigsäure:Sauerstoff:Ethylen 1 : (1,1 bis 1,3) : (0,75 bis 3) beträgt.

Es wurde nun gefunden, daß das Verhältnis von Essigsäure-konzentration zur Sauerstoffkonzentration im Reaktionsge-misch besonders kritisch ist und zwar in der Weise, daß bei einem molaren Essigsäure:Sauerstoff-Verhältnis von weniger als 2:1 exotherme Nebenreaktionen stattfinden, die zu höherem Abbrand, Leistungsabfall und Schädigung des Katalysators führen können. Beispielsweise wurde festgestellt, daß eine Herabsetzung des Essigsäure:Sauerstoff-Verhältnisses von ca. 3 : 1 auf ca. 1,6 : 1 einen sofortigen Leistungsabfall zur Folge hat, wobei sich auf dem Katalysator Kohle ablagert.

Gegenstand der Erfindung ist darum ein Verfahren zur Herstellung von Vinylacetat in der Gasphase durch Umsetzung von Ethylen, Essigsäure und molekularem Sauerstoff an palladiumhaltigen Trägerkatalysatoren, das dadurch gekennzeichnet ist, daß das molare Verhältnis von Essigsäure zu Sauerstoff im eingesetzten Gasgemisch oberhalb von 2 : 1 liegt. Vorzugsweise liegt das Verhältnis zwischen 2,2 : 1 und 3,5 : 1.

Zur Durchführung des erfindungsgemäßen Verfahrens wird das Ethylen, Essigsäure und Sauerstoff enthaltende Gasgemisch bei Temperaturen zwischen 100 und 220°C und bei Drücken zwischen 1 und 20 bar über den Katalysator geleitet, wobei nicht umgesetzte Komponenten im Kreise geführt werden können. Aus der Sicht einer wirtschaftlichen Aufarbeitung ist es zweckmäßig, die Essigsäurekonzentration im Reaktionsgemisch so zu wählen, daß ein möglichst hoher Vinylacetatgehalt im Rohkondensat und dementsprechend ein hoher Essigsäureumsatz erreicht wird. Unter Umständen ist auch eine Verdünnung des Reaktionsgemisches mit inerten Gasen wie Stickstoff oder Kohlendioxid vorteilhaft. Besonders Kohlendioxid eignet sich zur Verdünnung bei Kreisprozessen, da es in geringen Mengen als Nebenprodukt gebildet wird.

Das erfindungsgemäße Verfahren wird mit den bekannten, für die Acetoxylierung von Ethylen in Betracht kommenden Trägerkatalysatoren durchgeführt. Sie enthalten Palladium in elementarer Form und/oder als Verbindung in Form eines Komplexes oder eines Salzes, wie besonders Palladiumacetat.

Sie enthalten außerdem neben einem Alkaliacetat, wie besonders Kaliumacetat, verschiedene Aktivatoren, insbesondere Gold und/oder Cadmium in elementarer Form oder in Form von Verbindungen.

Als Katalysatorträger kommen alle inerten Stoffe in Betracht, die unter den Bedingungen der Acetoxylierung ihre mechanische Festigkeit nicht verlieren. Geeignet sind beispielsweise Kieselsäure, Kieselgel, Silikate, Alumosilikate, Aktivkohle, Aluminiumoxid, Spinelle, Zirkon, Bimsstein und Siliciumcarbid. Ein besonders geeignetes Trägermaterial ist z.B. eine Kieselsäure mit einer Oberfläche zwischen 40 und 300 $m^2$/g und einem mittleren Porenradius zwischen 50 und 2000 $\overset{o}{A}$.

Das erfindungsgemäße Verfahren zeichnet sich dadurch aus, daß man Vinylacetat in relativ hohen und vor allem in gleichbleibenden Raumzeitausbeuten herstellen kann, so daß es besonders wirtschaftlich ist.

Beispiel 1

Der Katalysator wird nach dem Verfahren der deutschen Patentschrift 1.667.053 durch Tränken eines Kieselsäureträgers mit einer Lösung von Palladiumacetat, Cadmiumacetat und Kaliumacetat in Essigsäure und Trocknen bei 50°C hergestellt. Er enthält 2 Gew.-% Palladium, 1,7 Gew.-% Cadmium und 1,9 Gew.-% Kalium. Über 1 Liter des Katalysators wird in einem ölbeheizten Reaktionsrohr von 30 mm lichter Weite bei einem Druck von 9 bar und einer Temperatur von 178°C ein Gasstrom von 4,5 $Nm^3$ pro Stunde geleitet, der sich aus 60,8 Vol.-% Ethylen, 17,4 Vol.-% Essigsäure und 6,3 Vol.-% Sauerstoff sowie als Rest Inertgas ($N_2$ + $CO_2$) zusammensetzt. Die Versuchsdauer beträgt ca. 350 Stunden. Die Reaktortemperatur bleibt während dieser Zeit konstant. Die Vinylacetat-Raumzeitausbeute beträgt 890 g/l·h. Sie ist nach 350 Stunden unverändert.

Vergleichsbeispiel 1

Der Versuch wird mit dem gleichen Katalysator unter gleichen Versuchsbedingungen wie im Beispiel 1 durchgeführt. Das Gasgemisch enthält neben Inertgas 61 Vol.-% Ethylen, 12,1 Vol.-% Essigsäure und 6,3 Vol.-% Sauerstoff. Es treten besonders am Reaktoreingang Temperaturschwankungen auf. Die Vinylacetat-Raumzeitausbeute beträgt nach 150 Stunden 865 g/l·h, nach 350 Stunden 815 g/l·h.

Beispiel 2

Der Katalysator wird nach dem im Beispiel 1 angegebenen Verfahren hergestellt. Er enthält 2,3 Gew.-% Palladium, 1,7 Gew.-% Cadmium, 1,9 Gew.-% Kalium und 0,07 Gew.-% Mangan. Der Versuch wird unter den im Beispiel 1 angegebenen Reaktionsbedingungen durchgeführt. Das Gasgemisch enthält neben Inertgas 61 Vol.-% Ethylen, 15 Vol.-% Essigsäure und 6 Vol.-% Sauerstoff. Die Reaktortemperatur bleibt während der ganzen Versuchsdauer (380 Stunden) konstant. Die Vinylacetat-Raumzeitausbeute beträgt 900 g/l·h.

Vergleichsbeispiel 2

Der Versuch wird zunächst genau so durchgeführt, wie im Beispiel 2 angegeben. Nach 150 Stunden wird die Essigsäurekonzentration im Reaktionsgemisch von 15 Vol.-% auf 11 Vol.-% herabgesetzt und die Inertgasmenge entsprechend vergrößert. Der Temperaturschreiber zeigt starke Temperaturschwankungen (hot spots) an. Nach 250 Stunden beträgt die Vinylacetat-Raumzeitausbeute 720 g/l·h.

Beispiel 3

Der Katalysator wird nach dem Verfahren der deutschen Patentschrift 2.509.251 durch Tränken eines Kieselsäureträgers mit Lösungen von Palladiumacetat und Kaliumacetat sowie von Bariumaurat in Essigsäure, Trocknen bei 60°C und 200 Torr unter Stickstoff und Reduktion mit einem Stickstoff-Wasserstoffgemisch hergestellt. Der Katalysator enthält 2 Gew.-% Palladium, 0.5 Gew.-% Gold und 1,9 Gew.-% Kalium. Über 1 Liter des Katalysators leitet man bei einem Druck von 9 bar und bei einer Temperatur von 175°C pro Stunde 4,5 Nm$^3$ eines Gasgemisches, das neben Inertgas 62 Vol.-% Ethylen, 17,8 Vol.-% Essigsäure und 5,9 Vol.-% Sauerstoff enthält. Während der Versuchszeit (300 Stunden) treten keinerlei Temperaturschwankungen auf. Die Vinylacetat-Raumzeitausbeute ist praktisch konstant. Sie beträgt 870 g/l·h.

Vergleichsbeispiel 3

Es wird zunächst so verfahren, wie im Beispiel 3 angegeben. Nach 120 Stunden wird die Essigsäurekonzentration im Reaktionsgemisch von 17,8 Vol.-% auf 9,5 Vol.-% verringert. Die Inertgaskonzentration wird in entsprechender Weise erhöht. Es treten sofort sehr starke Temperaturschwankungen auf, die nach etwa 5 Stunden abklingen. Die Vinylacetat-Raumzeitausbeute beträgt noch 450 g/l·h. Der Katalysator weist starke Kohleablagerungen auf, besonders der Teil, der sich in der Eingangszone des Reaktors befindet.

0012968

Patentansprüche:

1. Verfahren zur Herstellung von Vinylacetat in der Gasphase durch Umsetzung von Ethylen, Essigsäure und molekularem Sauerstoff an palladiumhaltigen Trägerkatalysatoren, dadurch gekennzeichnet, daß das molare Verhältnis von Essigsäure zu Sauerstoff im eingesetzten Gasgemisch oberhalb von 2 : 1 liegt.

2. Verfahren nach Anspruch 1, dadurch gekennzeichnet, daß das molare Verhältnis von Essigsäure zu Sauerstoff im eingesetzten Gasgemisch zwischen 2,2 : 1 und 3,5 : 1 liegt.

**Europäisches Patentamt**

# EUROPÄISCHER RECHERCHENBERICHT

0012968

Nummer der Anmeldung

EP 79 10 5213

| EINSCHLÄGIGE DOKUMENTE | | | KLASSIFIKATION DER ANMELDUNG (Int.Cl 3) |
|---|---|---|---|
| Kategorie | Kennzeichnung des Dokuments mit Angabe, soweit erforderlich, der maßgeblichen Teile | betrifft Anspruch | C 07 C 69/15 67/055 |
| X | <u>US - A - 3 634 496</u> (NAOYA KOMINAMI) <br> * Spalte 1, Zeilen 30-44; Spalten 5,6; Beispiele 3-18 * | 1,2 | |
| X | <u>FR - A - 1 371 111</u> (ASAHI KASEI) <br> * Seite 2, rechte Spalte, Absatz 4; Seite 3, Beispiele 1,3,4 * | 1,2 | |

**RECHERCHIERTE SACHGEBIETE (Int. Cl 3)**

C 07 C 69/15
67/055

**KATEGORIE DER GENANNTEN DOKUMENTE**

X: von besonderer Bedeutung

A: technologischer Hintergrund

O: nichtschriftliche Offenbarung

P: Zwischenliteratur

T. der Erfindung zugrunde liegende Theorien oder Grundsätze

E kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 24-03-1980 | KINZINGER |

EPA form 1503.1   06.78